(19) **Europäisches Patentamt / European Patent Office / Office européen des brevets**

(11) **EP 4 431 603 A1**

(12) # EUROPEAN PATENT APPLICATION
published in accordance with Art. 153(4) EPC

(43) Date of publication:
**18.09.2024 Bulletin 2024/38**

(21) Application number: **22892731.5**

(22) Date of filing: **07.11.2022**

(51) International Patent Classification (IPC):
**C12N 15/10** (2006.01)   **C12Q 1/6806** (2018.01)
**C40B 40/06** (2006.01)

(52) Cooperative Patent Classification (CPC):
**C12N 15/10; C12Q 1/6806; C40B 40/06**

(86) International application number:
**PCT/JP2022/041394**

(87) International publication number:
**WO 2023/085232 (19.05.2023 Gazette 2023/20)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **10.11.2021   JP 2021183612**

(71) Applicants:
• **TOYOBO CO., LTD.**
  **Osaka-shi, Osaka 530-0001 (JP)**
• **RIKEN**
  **Wako-shi, Saitama 351-0198 (JP)**

(72) Inventors:
• **YAMAKOSHI, Nana**
  **Tsuruga-shi, Fukui 914-8550 (JP)**
• **TAKEDA, Taka-aki**
  **Tsuruga-shi, Fukui 914-8550 (JP)**
• **WATANABE, Satomi**
  **Tsuruga-shi, Fukui 914-8550 (JP)**
• **HAYASHI, Tetsutaro**
  **Wako-shi, Saitama 351-0198 (JP)**
• **NIKAIDO, Itoshi**
  **Wako-shi, Saitama 351-0198 (JP)**

(74) Representative: **Vossius & Partner**
**Patentanwälte Rechtsanwälte mbB**
**Siebertstrasse 3**
**81675 München (DE)**

(54) **IMPROVED LIBRARY PREPARATION METHOD**

(57)   Provided is a method for efficiently preparing a library.

Disclosed is a method for preparing a library, comprising the following steps (a), (b), and (c):
(a) synthesizing single-stranded cDNA from 10 pg or more of template RNA;
(b) synthesizing double-stranded cDNA from the single-stranded cDNA; and
(c) preparing a library using the double-stranded cDNA that is unpurified.

EP 4 431 603 A1

**Description**

Technical Field

The present invention relates to a method for preparing a library.

Background Art

In nucleic acid sequence analysis of DNA, RNA, etc., next-generation sequencing (NGS) technology, which enables vast amounts of nucleotide sequence data to be obtained in a short period, has advanced rapidly. Next-generation sequencing is a powerful analysis technique that contributes to the field of life science, such as medical technology (e.g., clinical diagnosis and drug development), agricultural technology, and basic research, because of its advanced and high-speed processing that can sequence multiple individuals simultaneously.

In next-generation sequencing, it is usually necessary to prepare a library composed of nucleic acid fragments with adapter sequences (which can function as, for example, sites for binding to a flow cell and primer binding sites) added to their ends.

When a library is prepared using RNA as a template, a method comprising performing library preparation after purification of the generated double-stranded cDNA solution is used. For example, the RT-RamDA method is a reverse transcription method with random displacement amplification in which cDNA is amplified using RNA as a template by incubating a mixture comprising template RNA, a primer, a degrading enzyme specific to DNA strand in RNA-DNA hybrid strand, an RNase H minus reverse transcriptase, and a substrate; in preparing a library, double-stranded cDNA is generated from single-stranded cDNA, the resulting solution is purified, and then library preparation is started (Patent Literature (PTL) 1 and Non-patent Literature (NPL) 1).

Citation List

Patent Literature

PTL 1: WO2016/052619

Non-patent Literature

NPL 1: Hayashi T. et al., Single-cell full-length total RNA sequencing uncovers dynamics of recursive splicing and enhancer RNAs, Nat. Commun., 2018

Summary of Invention

Technical Problem

Double-stranded cDNA solutions contain cell-derived contaminants, enzymes used in reverse transcription reaction, primers, dNTPs, salts, etc., which can interfere with the reaction in the subsequent step. In particular, these substances in the solutions can significantly interfere with the enzyme reaction used in library preparation. However, the purification operation is complicated, and its workability is poor. The purification step also reduces the yield. For these reasons, it is desirable to simplify the purification step. A primary object of the present invention is to provide a method for more efficiently preparing a library.

Solution to Problem

The present inventors conducted extensive research to achieve the above object and found that even with a small amount of template RNA, a sufficient library yield can be obtained by directly carrying the generated double-stranded cDNA to library preparation, without purification. The present invention has been accomplished as a result of further research based on this finding.

The present invention typically includes the following subject matter.

[Item 1]

A method for preparing a library, comprising the following steps (a), (b), and (c):

(a) synthesizing single-stranded cDNA from 10 pg or more of template RNA;
(b) synthesizing double-stranded cDNA from the single-stranded cDNA; and
(c) preparing a library using the double-stranded cDNA that is unpurified.

[Item 2]

The method according to Item 1, wherein step (b) is performed in the presence of 12 mM or less of sodium chloride.

[Item 3]

The method according to Item 1 or 2, wherein step (a) is performed in the presence of 1 ng/$\mu$L or more of a nucleic acid polymer and/or 1 U/mL or more of a proteolytic enzyme.

[Item 4]

The method according to any one of claims 1 to 3, further comprising heating the double-stranded cDNA at 80°C or higher for 10 minutes or more.

[Item 5]

The method according to Item 3 or 4, wherein the nucleic acid polymer is at least one homopolymer selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof.

[Item 6]

The method according to any one of items 3 to 5, wherein the nucleic acid polymer is at least one homopolymer selected from the group consisting of polyinosinic acid, polydeoxyinosinic acid, and salts thereof.

[Item 7]

The method according to any one of Items 3 to 6, wherein the proteolytic enzyme comprises any of proteinase K and subtilisin.

[Item 8]

The method according to any one of Items 1 to 7, wherein the template RNA is RNA extracted from 1 to 1000 cells.

[Item 9]

The method according to any one of Items 1 to 8, wherein the double-stranded cDNA that is unpurified of step (c) is in the form of 1 $\mu$L or less of a solution.

[Item 10]

The method according to any one of Items 1 to 9, wherein step (a) is performed by an RT-RamDA method.

[Item 11]

The method according to any one of Items 1 to 10, wherein step (b) is performed using a Klenow fragment.

[Item 12]

The method according to any one of Items 1 to 11, wherein step (c) is performed by any of a transposon method and a ligation method.

[Item 13]

The method according to any one of Items 1 to 12, wherein step (b) is performed in the presence of 60 mM or less of a chloride.

Advantageous Effects of Invention

The present invention provides a method for efficiently preparing a library using, for example, unpurified double-stranded cDNA. In particular, the present invention provides a method for preparing a library in a sufficient yield without purifying double-stranded cDNA generated, even from a small amount (about 10 pg) of template RNA. The method is useful for library preparation for next-generation sequencing. The method allows a library to be efficiently prepared even when the amount of double-stranded cDNA solution used is 1 μL or less, which is much smaller than the amount used in conventional library preparation methods. Thus, the amount of reagents required for library preparation can be reduced, resulting in cost reduction. It is usually the case that the amount of double-stranded cDNA solution synthesized from template RNA is larger than 1 μL. However, by using 1 μL or less of a double-stranded cDNA solution for library preparation, the excess amount of the double-stranded cDNA solution can be used for other applications or quality checks. Use for such applications or quality checks is beneficial because it makes it possible to determine whether to proceed to the next step, which saves time, labor, and materials.

Brief Description of Drawings

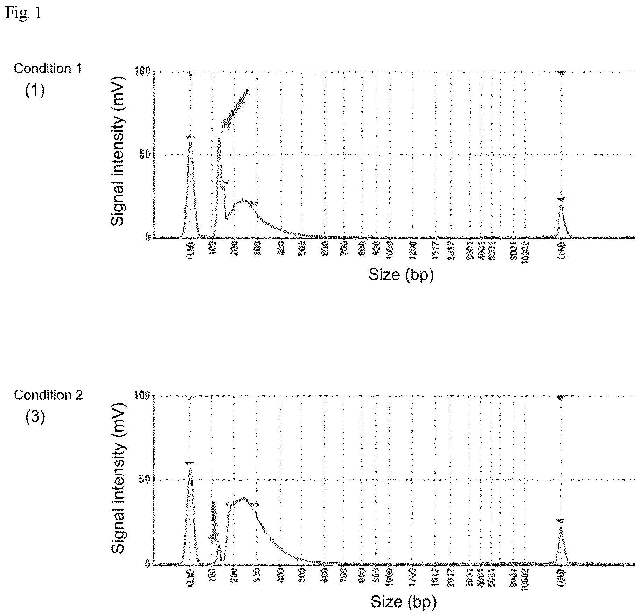

Fig. 1 shows the results of measuring libraries with MultiNA (Shimadzu Corporation) in Example 5.

Description of Embodiments

The present invention is described in more detail below while showing embodiments of the present invention.

Method for Preparing Library

In one embodiment, the method for preparing a library preferably comprises the following steps (a), (b), and (c):

(a) synthesizing single-stranded cDNA from 10 pg or more of template RNA;
(b) synthesizing double-stranded cDNA from the single-stranded cDNA; and
(c) preparing a library using the double-stranded cDNA that is unpurified.

1. Step (a)

The template RNA can be any RNA such as RNA extracted from tissue or single or more cells (extracted RNA may be RNA further purified (e.g., purified RNA treated with any purification technique known in the art, such as ethanol precipitation or column purification). The type of cell from which template RNA is extracted is not particularly limited, and any type of cell may be used. The amount of the template RNA is preferably 10 pg or more. The upper limit of the amount of the template RNA is not particularly limited, and is preferably 10 ng or less, 5 ng or less, 1 ng or less, 500 pg or less, or 100 pg or less. In the present invention, even if the amount of the template RNA is small (e.g., 10 pg or more and 1 ng or less), a library can be efficiently prepared from the obtained double-stranded cDNA. The amount of the template RNA may be, for example, the amount contained in a small number of cells (e.g., 1 to 1000 cells, preferably 1 to 100 cells, more preferably 1 to 10 cells, and more preferably 1 cell). That is, RNA extracted from the above number of cells can be used as the template RNA. It is said that the standard amount of RNA contained in single cell is about 10 pg. The present invention makes it possible to prepare a library from a small amount of the template RNA, thus enabling a library to be prepared in a sufficient yield even from single cell.

In one embodiment, the template RNA is preferably RNA extracted by lysing single or more cells by using a composition for cell lysis.

The composition for cell lysis contains at least a cell lysis agent. Examples of cell lysis agents include surfactants, chaotropic agents, and the like. Examples of surfactants include anionic surfactants (e.g., sodium dodecyl sulfate, sodium cholate, and sodium deoxycholate), cationic surfactants (e.g., cetyltrimethylammonium bromide), nonionic surfactants (e.g., octylphenol ethoxylate, polyoxyethylene lauryl ether, polyoxyethylene stearyl ether, and polyoxyethylene sorbitan monolaurate), and zwitterionic surfactants (e.g., 3-[(3-cholamidopropyl)dimethylammonio]-1-propanesulfonic acid). Examples of chaotropic agents include urea and lithium salts such as lithium perchlorate. The cell lysis agent is typically dissolved in water (preferably nuclease-free water) and used in the form of an aqueous solution. The cell lysis agents may be used singly or in a combination of two or more.

The composition for cell lysis may contain an additional component. Examples of additional components include proteolytic enzymes (e.g., proteinase K and subtilisin), nucleic acid polymers, RNase inhibitors, and combinations of two or more thereof.

The nucleic acid polymer is preferably at least one member selected from the group consisting of inosinic acid polymers, cytidylic acid polymers, guanylic acid polymers, adenylic acid polymers, thymidylic acid polymers, uridylic acid polymers, deoxyinosinic acid polymers, deoxycytidylic acid polymers, deoxyguanylic acid polymers, deoxyadenylic acid polymers, deoxythymidylic acid polymers, and deoxyuridylic acid polymers. For example, "inosinic acid polymers" used here encompass inosinic acid homopolymers (polyinosinic acid), inosinic acid copolymers (e.g., copolymers containing a structural unit derived from inosinic acid in an amount of more than 50 mol%, 60 mol% or more, 70 mol% or more, 80 mol% or more, or 90 mol% or more, and less than 100 mol%), derivatives thereof (e.g., those in which a functional group, such as a fluorine atom, a bromine atom, an iodine atom, an alkyl group, an amino group, or a mercapto group, is introduced into at least part of the base moieties and/or those in which at least part of the phosphate moieties is replaced by a thiophosphate moiety), and salts thereof (e.g., alkali metal salts, such as sodium salts and potassium salts), as well as a double-stranded polymer referred to as, for example, "poly(I:C)" formed by annealing polyinosinic acid and polycytidylic acid. The same applies to the other nucleic acid polymers, including cytidylic acid polymers.

In one embodiment, the nucleic acid polymer is preferably at least one member selected from the group consisting of inosinic acid polymers, cytidylic acid polymers, guanylic acid polymers, deoxyinosinic acid polymers, deoxycytidylic acid polymers, and deoxyguanylic acid polymers, and more preferably an inosinic acid polymer and/or a deoxyinosinic acid polymer.

In one embodiment, the nucleic acid polymer is preferably at least one homopolymer selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof, and more preferably at least one homopolymer selected from the group consisting of polyinosinic acid, polydeoxyinosinic acid, and salts thereof.

The nucleic acid polymer may have any length. In an example, the nucleic acid polymer may have a total length of 30 to 10000 bases.

Step (a) is preferably performed in the presence of a proteolytic enzyme and/or a nucleic acid polymer. Synthesizing single-stranded cDNA in the presence of these components can increase the library yield. This is believed to be partly because these components alleviate inhibition of library preparation caused by cell-derived contaminants and substances such as a reverse transcriptase used in reverse transcription reaction.

When a proteolytic enzyme and/or a nucleic acid polymer is contained in the composition for cell lysis that can be used to prepare template RNA, the reaction solution does not have to contain a proteolytic enzyme and/or a nucleic acid polymer.

The concentration of the proteolytic enzyme in the synthesis reaction solution of step (a) is preferably 0.001 mg/mL or more. The concentration of the proteolytic enzyme in the synthesis reaction solution in step (a) is, for example, 0.001 to 0.1 mg/mL, preferably 0.001 to 0.01 mg/mL, and more preferably 0.001 to 0.005 mg/mL. The concentration of the proteolytic enzyme in the synthesis reaction solution of step (a) can also be expressed in activity units, and is preferably 1 U/mL or more. The concentration of the proteolytic enzyme in the synthesis reaction solution of step (a) is, for example, 1 U/mL to 100 U/mL, preferably 1 U/mL to 10 U/mL, and more preferably 1 U/mL to 5 U/mL.

The activity of the proteolytic enzyme is determined by the definitions commonly used in the art. For example, in the definition of the activity of proteinase K, the enzyme activity that causes an increase in optical density at 275 nm corresponding to 1 $\mu$g of tyrosine per minute under the conditions described below is defined as 1 unit (1 U). The activity of other proteolytic enzymes such as subtilisin can also be measured in the same manner.

Reagents

(A) Casein solution (substrate solution): 2.0% (20.0 g of Hammarsten milk casein (Merck) is suspended and dissolved in 400 mL of a 0.5% NaOH solution. The pH is adjusted to 7.2 with 1 N HCl. 500 mL of a 20 mM borate buffer containing 4 mM $CaSO_4$ (pH 7.2) is added, and water is added to make a total volume of 1000 mL).
(B) TCA solution: 20% trichloroacetic acid (TCA)
(C) Enzyme diluent: 10 mM borate buffer containing 2 mM $CaSO_4$ (pH 8.0)

Concentration in assay mixture

Borate buffer: 10 mM
Casein: 1.0%
$CaSO_4$: 2 mM

Procedure

1. 1.0 mL of the substrate solution (A) and 0.9 mL of the enzyme diluent (C) are pipetted into a test tube and equilibrated at 35°C for about 5 minutes.
2. 0.1 mL of a sample to be measured is added, followed by mixing.
3. After 10 minutes at 35°C, 2.0 mL of the TCA solution (B) is added to stop the reaction.
4. Incubation is performed at 35°C for another 30 minutes.
5. Centrifugation is performed at 14000 rpm, and the absorbance of the supernatant at 275 nm is measured (OD test).

At the same time, after incubation at 35°C for 10 minutes, a blank is prepared by first mixing the substrate solution with 2.0 mL of the TCA solution (B) and then adding a sample to be measured, and the same steps 4-5 are performed (OD blank) .
The activity is calculated using the following formulas.

$$\text{Volume activity (U/mL)} = \{OD(OD\ test\text{-}OD\ blank) \times Vt \times df\} / \{0.0074 \times 1.0 \times t \times Vs\} = OD \times 541 \times df$$

$$\text{Weight activity (U/mg)} = (U/ml) \times 1/C$$

Vt: total volume (4.0 mL)
Vs: sample volume (0.1 mL)
0.0074: extinction coefficient of tyrosine ($cm^2/\mu g$)
t: reaction time (10 minutes)
df: dilution factor
C: Enzyme concentration during dissolution (mg/mL)

When proteinase K (Promega Corporation) is measured by the above method, a 0.001 mg/mL solution corresponds to about 1 U/mL (1.2 to 1.4 U/mL).
The concentration of the nucleic acid polymer in the synthesis reaction solution of step (a) is preferably 1 ng/$\mu$L or more. The concentration of the nucleic acid polymer in the synthesis reaction solution of step (a) is, for example, 1 to 10 ng/uL, preferably 1 to 5 ng/$\mu$L, and more preferably 1 to 2 ng/$\mu$L.
The reaction for synthesizing single-stranded cDNA from template RNA is not particularly limited, and various conventionally known methods can be used.

1-1. Reverse Transcription Reaction

One example of the reaction for synthesizing single-stranded cDNA from template RNA is reverse transcription reaction. When step (a) is performed by reverse transcription reaction, it typically includes the step of incubating a composition for reverse transcription (or a solution for reverse transcription reaction) containing a protein having reverse transcription activity such as a reverse transcriptase.
The composition for reverse transcription contains, for example, template RNA, a proteolytic enzyme and/or nucleic acid polymer, a primer, a deoxyribonucleotide, and a reverse transcriptase. The composition for reverse transcription may optionally contain other components, such as a DNA polymerase.
The primer includes, for example, a primer specific to template RNA, an oligo-dT primer, a random primer, and a combination of two or more thereof. The present invention is beneficial in performing reverse transcription reaction by using an oligo-dT primer and/or a random primer, and is particularly beneficial in performing reverse transcription reaction by using a combination of an oligo-dT primer and a random primer. The molar ratio of the oligo-dT primer to the random primer is, for example, 1:5 to 1:15, and preferably 1:8 to 1:12.
Examples of random primers include completely random primers and NSR (not so random) primers.
The completely random primers refer to a mixture of primers of various base sequences, in which each base sequence is completely random. The completely random primers may contain a completely identical sequence (or completely complementary) to the sequence of rRNA. Examples of completely random primers include completely random pentamers, completely random hexamers, completely random heptamers, completely random octamers, and combinations thereof. For example, completely random hexamers may be a mixture of all possible base sequences formed by the four nucleotides (A, T, C, and G) (46 types).

The NSR primers refers to primers prepared by removing a primer whose sequence is completely complementary to the sequence of rRNA from completely random primers. Examples of the sequence of rRNA to be removed include the sequence of 18S rRNA, the sequence of 28S rRNA, the sequence of 12S rRNA, the sequence of 16S rRNA, and combinations thereof.

Examples of NSR primers include those prepared by removing a hexamer with a sequence completely complementary to the sequence of rRNA from completely random hexamers. Those prepared by removing one with a sequence completely complementary to the sequence of rRNA from a primer set such as of completely random pentamers, completely random heptamers, and completely random octamers are also usable as NSR primers.

From the standpoint of annealing, the length of the primers is, for example, 5 bases or more, and preferably 6 bases or more; from the standpoint of synthesis, the length of the primers is, for example, 30 bases or less, preferably 25 bases or less, or more preferably 20 bases or less.

The primers in the composition for reverse transcription can be of any concentration; the concentration of the primers is, for example, 1 to 10 $\mu$M, preferably 2 to 6 $\mu$M, or more preferably 3 to 5 $\mu$M.

The deoxyribonucleotide is preferably deoxyribonucleoside triphosphate. Examples of deoxyribonucleoside triphosphate include deoxycytidine triphosphate (dCTP), deoxyguanosine triphosphate (dGTP), deoxyadenosine triphosphate (dATP), deoxythymidine triphosphate (dTTP), deoxyuridine triphosphate (dUTP), derivatives thereof, and combinations of two or more thereof. Of these, for example, a mixture of dCTP, dGTP, dATP, and dTTP, a mixture of dCTP, dGTP, dATP, and dUTP, and a mixture of dCTP, dGTP, dATP, dTTP, and dUTP are preferable.

The reverse transcriptase refers to any protein (enzyme) that has reverse transcription activity (RNA-dependent DNA polymerase activity); the reverse transcriptase can be any reverse transcriptase and is preferably a polymerase that has reverse transcriptase activity. The reverse transcriptase is preferably one that has low RNase H activity or no RNase H activity. Examples of reverse transcriptases include avian myeloblastosis virus reverse transcriptase (AMV-RT), Moloney murine leukemia virus reverse transcriptase (MMLV-RT), human immunovirus reverse transcriptase (HIV-RT), EIRV-RT, RAV2-RT, C. *hydrogenoformans* DNA polymerase, rTthDNA polymerase, SuperScript I, SuperScript II, variants thereof, and derivatives thereof. Of these, MMLV-RT is preferable.

The composition for reverse transcription may contain the following DNA polymerases or contain no such DNA polymerases: Taq, Tbr, Tfl, Tru, Tth, Tli, Tac, Tne, Tma, Tih, Tfi, Pfu, Pwo, Kod, Bst, Sac, Sso, Poc, Pab, Mth, Pho, ES4, VENT (trademark), DEEPVENT (trademark), and variants thereof.

The composition for reverse transcription may contain an RNase inhibitor. The RNase inhibitor can be any RNase inhibitor. Examples of RNase inhibitors include human placenta-derived proteins, rat lung-derived proteins, and swine liver-derived proteins.

The composition for reverse transcription may contain additives. Examples of additives include buffers, salts, and combinations of two or more thereof.

Examples of buffers include Tris, Tricine, Bis-Tricine, Hepes, Mops, Tes, Taps, Pipes, Caps, and combinations of two or more thereof. The buffer is typically dissolved in water (preferably nuclease-free water) and used in the form of an aqueous solution.

Examples of salts include chlorides (e.g., lithium chloride, sodium chloride, potassium chloride, magnesium chloride, and manganese chloride), acetates (e.g., lithium acetate, sodium acetate, potassium acetate, magnesium acetate, and manganese acetate), sulfates (e.g., potassium sulfate, magnesium sulfate, and manganese sulfate), and combinations of two or more thereof.

The conditions for incubating the composition for reverse transcription are not particularly limited as long as single-stranded cDNA is generated from template RNA, and any conditions known in the art can be applied. The temperature of incubation is, for example, 30 to 65°C, and preferably 35 to 60°C. The time period for incubation is, for example, 5 to 120 minutes, and preferably 10 to 60 minutes.

1-2. Reverse Transcription Method with Random Displacement Amplification to Amplify cDNA Using RNA as Template (also called "RT-RamDA method")

The RT-RamDA method refers to a nucleic acid amplification method that includes the step of incubating a mixture that contains template RNA, a primer, a degrading enzyme specific to DNA strand in RNA:DNA hybrid strand, an RNase H minus reverse transcriptase, and a substrate. In the RT-RamDA method, due to RNA-dependent DNA polymerase activity of the RNase H minus reverse transcriptase, complementary strand DNA (cDNA) of the template RNA is synthesized, and the cDNA strand of the hybrid strand of RNA and cDNA is randomly cleaved by the degrading enzyme specific to DNA strand in RNA:DNA hybrid strand. With the cleavage site as a starting point, the cDNA strand at the 3' side is peeled off from RNA due to strand displacement activity of the RNase H minus reverse transcriptase, and a new cDNA strand is synthesized at the portion peeled off by the RNase H minus reverse transcriptase. The details of the RT-RamDA method are disclosed, for example, in US Patent Application Publication No. 2017/0275685, the entire disclosure of which is incorporated herein by reference.

When step (a) is performed by the RT-RamDA method, it includes the step of incubating the composition for reverse transcription that contains a degrading enzyme specific to DNA strand in RNA:DNA hybrid strand. The degrading enzyme specific to DNA strand in RNA:DNA hybrid strand preferably has activity of cleaving the DNA strand in a hybrid strand of RNA and DNA. The enzyme for use may be, for example, a double strand-specific DNA degrading enzyme or a non-specific DNA degrading enzyme. Of these, a non-specific DNA degrading enzyme is preferable in the present invention.

The double strand-specific DNA degrading enzyme (double-strand-specific nuclease; also called "DSN") may be an enzyme derived from a prokaryote or a eukaryote and is preferably a double strand-specific DNA degrading enzyme derived from a crustacean or a modified product thereof. Specific examples include the following:

- *Solenocera melantho* DNase
- *Penaeus japonicus* DNase
- *Paralithodes camtschaticus* DSN
- *Pandalus borealis* dsDNase
- *Chionoecetes opilio* DSN
- Other DSN homologs

The double strand-specific DNA degrading enzyme preferably has DNA-degrading activity even at a temperature below 60°C. Of the DNA degrading enzymes above, double strand-specific DNA degrading enzymes derived from a prawn or shrimp or modified products thereof are preferable.

The double strand-specific DNA degrading enzyme for use can be a commercially available product. Examples of commercially available products of double strand-specific DNA degrading enzymes include dsDNase (ArcticZymes), HI-dsDNase (ArcticZymes), dsDNase (Thermo Scientific), Shrimp Dnase, Recombinant (Affymetrix), Atlantis dsDNase (Zymo Research), Thermolabile Nuclease (Roche), and the like.

The non-specific DNA degrading enzyme is, for example, an enzyme that has activity of cleaving the DNA strand of a hybrid strand of RNA and DNA, and substantially has no activity of cleaving the RNA strand of a hybrid strand of RNA and DNA and single-stranded RNA, and preferably an enzyme that has activity of cleaving single-stranded DNA lower than the activity of cleaving the DNA strand of a hybrid strand of RNA and DNA. The non-specific DNA degrading enzyme preferably has DNA-degrading activity even at a temperature below 60°C. The non-specific DNA degrading enzyme for use may be a commercially available product. Examples of non-specific DNA degrading enzymes for use include DNase I (DNase I produced by Thermo Fisher Scientific) and the like.

The non-specific DNA degrading enzyme for use may be an enzyme derived from a prokaryote or a eukaryote and is preferably a non-specific DNA degrading enzyme derived from a mammal or a modified product thereof, and more preferably a bovine-derived non-specific DNA degrading enzyme or a modified product thereof.

The "modified product" above refers to an enzyme obtained by modifying a naturally occurring amino acid sequence. Specifically, a modified product refers to an enzyme having an amino acid sequence that is at least 80% (preferably at least 90%, more preferably at least 95%) identical to a naturally occurring amino acid sequence, and an enzyme having an amino acid sequence that contains one or a few (e.g., 1 to 10, preferably 1 to 5, more preferably 1 to 3) deletions, substitutions, and/or additions of amino acids compared with a neutrally occurring amino acid sequence.

When step (a) is performed by the RT-RamDA method, the composition for reverse transcription may contain a single-stranded-DNA binding protein. The single-stranded-DNA binding protein is typically used together with a degrading enzyme specific to DNA strand in RNA:DNA hybrid strand. Examples of single-stranded-DNA binding proteins include T4 gene 32 protein, RecA, SSB (Single-Stranded DNA Binding Protein), and combinations of two or more thereof.

When step (a) is performed by the RT-RamDA method, the composition for reverse transcription may be incubated under isothermal conditions or under thermal cycling conditions.

(1) Isothermal Conditions

Incubation under isothermal conditions can be performed, for example, at a predetermined temperature of 25°C or higher and below 50°C, preferably at a predetermined temperature between 30 and 45°C, and more preferably at a predetermined temperature between 35 and 40°C, for example, at 37°C for a predetermined period of time (e.g., 5 to 180 minutes, preferably 10 to 150 minutes).

Incubation at a predetermined temperature of 25°C or higher and below 50°C may be performed in two or more steps. For example, incubation can be performed at a predetermined temperature of 25°C or higher and below 30°C for 5 to 15 minutes, and then performed at a predetermined temperature of 30°C or higher and below 35°C for 5 to 15 minutes, followed by incubation at a predetermined temperature of 35°C or higher and below 50°C for a predetermined period of time (e.g., 5 to 60 minutes).

After incubation at a predetermined temperature of 25°C or higher and below 50°C, incubation may be performed, for example, at a predetermined temperature of 50°C or higher and below 100°C. The incubation at a predetermined temperature of 50°C or higher and below 100°C may be performed in two or more steps. For example, incubation may be performed at a predetermined temperature of 50°C or higher and below 80°C for 5 to 15 minutes, and then performed at a predetermined temperature between 80 to 100°C for 5 to 15 minutes.

(2) Thermal Cycling Conditions

Incubation under thermal cycling conditions can be performed, for example, at predetermined temperature T1 that is 20°C or higher and below 30°C (e.g., 25°C) and at predetermined temperature T2 that is between 30 and 45°C (e.g., 37°C) in combination for a predetermined period of time for T1 (e.g., 1 to 3 minutes; 2 minutes, for example) and for a predetermined period of time for T2 (e.g., 1 to 3 minutes; 2 minutes, for example). This operation can be taken as one cycle and may be performed preferably 10 to 40 cycles, and more preferably 15 to 35 cycles. Before the thermal cycling, incubation may be performed, for example, at a predetermined temperature of 25°C or higher and below 30°C for a predetermined period of time (e.g., 5 to 15 minutes) and performed at a predetermined temperature of 30°C or higher and below 35°C for a predetermined period of time (e.g., 5 to 15 minutes), followed by incubation at a predetermined temperature of 35°C or higher and below 50°C for a predetermined period of time (e.g., 1 to 5 minutes). After the above thermal cycling, incubation may be performed, for example, at a predetermined temperature of 50°C or higher and below 80°C for a predetermined period of time (e.g., 5 to 15 minutes) and performed at a predetermined temperature between 80 and 90°C for a predetermined period of time (e.g., 5 to 15 minutes).

2. Step (b)

Step (b) is preferably performed in the presence of a salt. Synthesizing double-stranded cDNA in the presence of a salt can further increase the library yield. This is believed to be partly because of an increase in generated double-stranded cDNA due to optimized binding of the primer to the template.

Examples of salts include chlorides (e.g., alkali metal chlorides, such as lithium chloride, sodium chloride, and potassium chloride; alkaline earth metal chlorides, such as magnesium chloride; and transition metal chlorides, such as manganese chloride). Of these, alkali metal chlorides and/or alkaline earth metal chlorides are preferable, and at least one member selected from the group consisting of magnesium chloride, potassium chloride, and sodium chloride is preferable. The salts may be used singly or in a combination of two or more.

When the reaction product of step (a) contains a salt, a salt may not be added to the reaction solution of step (b) or may be further added to the reaction solution of step (b).

The concentration of the salt(s) in the synthesis reaction solution of step (b) is, for example, 60 mM or less, preferably 50 mM or less, and more preferably 40 mM or less. The concentration of the salt(s) in the synthesis reaction solution of step (b) is also, for example, 5 to 60 mM, preferably 5 to 50 mM, and more preferably 5 to 40 mM. In a specific embodiment, the concentration of the salt(s) in the synthesis reaction solution of step (b) is, for example, 20 mM or less (e.g., 5 to 20 mM), 15 mM or less (e.g., 5 to 15 mM) or 12 mM or less (e.g., 5 to 12 mM). The concentration of the salt(s) is preferably the concentration of chloride(s).

Among the salt(s) in the synthesis reaction solution of step (b), the concentration of sodium chloride is preferably lower. The concentration of sodium chloride in the synthesis reaction solution of step (b) is, for example, 20 mM or less, preferably 15 mM or less, and more preferably 12 mM or less. The synthesis reaction solution of step (b) does not have to contain sodium chloride; or when the synthesis reaction solution of step (b) contains sodium chloride, the concentration of sodium chloride in the synthesis reaction solution of step (b) is, for example, 5 to 20 mM, preferably 5 to 15 mM, and more preferably 5 to 12 mM.

Among the salt(s) in the synthesis reaction solution of step (b), the concentration of potassium chloride is, for example, 5 mM or more (e.g., 5 to 50 mM), preferably 10 mM or more (e.g., 10 to 40 mM), and more preferably 15 mM or more (e.g., 15 to 35 mM).

Among the salt(s) in the synthesis reaction solution of step (b), the concentration of magnesium chloride is, for example, 20 mM or less (e.g., 1 to 20 mM), preferably 15 mM or less (e.g., 1 to 15 mM), and more preferably 10 mM or less (e.g., 1 to 10 mM).

In a specific embodiment, in the synthesis reaction solution of step (b), it is preferred that the concentration of chloride(s) is 60 mM or less (e.g., 50 mM or less, preferably 40 mM or less) and that the concentration of sodium chloride is 20 mM or less; and it is more preferred that the concentration of chloride(s) is 60 mM or less (e.g., 50 mM or less, preferably 40 mM or less), that the concentration of sodium chloride is 20 mM or less, and that the concentration of potassium chloride is 5 mM or more.

The reaction for synthesizing double-stranded cDNA from single-stranded cDNA is not particularly limited, and various conventionally known methods can be used. The reaction typically involves incubating a composition for double-stranded cDNA synthesis containing an enzyme (double-stranded cDNA synthesis enzyme) that catalyzes a polymerization reaction of nucleotides in the 5' to 3' direction in the presence of template DNA and a primer.

The composition for double-stranded cDNA synthesis may contain, for example, single-stranded cDNA, a salt, a primer, a deoxyribonucleotide, and a double-stranded cDNA synthesis enzyme. The composition for double-stranded cDNA synthesis may optionally contain other components, such as a buffer.

The double-stranded cDNA synthesis enzyme is an enzyme that catalyzes a polymerization reaction of nucleotides in the 5' to 3' direction and is not particularly limited. Examples include Klenow fragments, T4 DNA polymerase, variants thereof, and derivatives thereof. Of these, Klenow fragments are preferable.

Examples of primers, deoxyribonucleotides, and buffers include those mentioned in the section regarding the composition for reverse transcription described above.

The conditions for incubating the composition for double-stranded cDNA synthesis are not particularly limited as long as double-stranded cDNA is generated from single-stranded cDNA, and any conditions known in the art can be applied. For example, incubation may be performed at a predetermined temperature of 10°C or higher and below 35°C for a predetermined period (e.g., 5 to 90 minutes).

In one embodiment, it is preferred that the method for preparing a library further comprises the step of heating the double-stranded cDNA (e.g., the reaction solution of step (b)). This step is preferably performed after step (b) and before step (c). The step can correspond to inactivation treatment. The heating conditions (conditions for inactivation) are not particularly limited. For example, incubation may be performed at a predetermined temperature of 70°C or higher (preferably 75°C or higher, more preferably 80°C or higher) for a predetermined period (e.g., 5 minutes or more, preferably 10 minutes or more). The heating temperature (temperature for inactivation) is preferably 80°C or higher and 95°C or lower, more preferably 80°C or higher and 90°C or lower, and even more preferably 80°C or higher and 85°C or lower. The incubation time is preferably 5 minutes or more and 30 minutes or less, more preferably 10 minutes or more and 20 minutes or less, and even more preferably 10 minutes or more and 15 minutes or less. By performing such a heating step, the formation of adapter dimers can be more effectively suppressed in step (c) . Without being bound by theory, heating at such a temperature presumably unfolds the higher-order structure of the double-stranded cDNA to improve enzyme reaction efficiency (e.g., transposon reaction) in library preparation, thereby more effectively suppressing the formation of adapter dimers.

### 3. Step (c)

The library preferably comprises nucleic acid fragments to which sequences necessary for next-generation sequencing are added. The sequences necessary for next-generation sequencing include flow cell binding sequences and sequences necessary for sequencing.

The next-generation sequencing (also referred to as "NGS analysis," "next-generation sequencer analysis," etc.) as used herein typically refers to a sequencing technique capable of simultaneously performing tremendous sequencing reactions of millions to billions. Examples of next-generation sequencing include methods for performing sequence analysis in parallel by using an amplification technique such as emulsion PCR and bridge PCR and a high-sensitivity detection technique such as one-molecule observation. Examples of apparatuses for performing next-generation sequencing (sequencers) include, but are not particularly limited to, MiSeq, HiSeq, and NovaSeq (Illumina, Inc.); Genetic Analyzer V2.0 and Ion Proton (Thermo Fisher Scientific, Inc.); MinION and PromethION (Nanopore); and the like. Next-generation sequencing is disclosed in, for example, US Patent Application Publication No. 2014/178438, the entire disclosure of which is incorporated herein by reference.

The double-stranded cDNA used for library preparation is, for example, in the form of 5 μL or less, preferably 4 μL or less, more preferably 3 μL or less, even more preferably 2 μL or less, and particularly preferably 1 μL or less, of a solution. The double-stranded cDNA is, for example, in the form of 0.01 μL or more, 0.05 μL or more, or 0.1 μL or more, of a solution. In the present invention, a sufficient library yield can be obtained without purification even with a small amount of double-stranded cDNA.

The method for preparing the library is not particularly limited. For example, template DNA is fragmented and tagged using a transposon, and nucleic acid amplification of a library composed of tagged DNA fragments is performed. For example, two transposon end sequences (corresponding to adapters) and a transposase form a transposome complex. The transposome complex fragments and tags template DNA in a solution, and the sequences necessary for next-generation sequencing are added by PCR or the like, thereby enabling sequence analysis (this method may be referred to as a "transposon method"). Commercially available kits using a transposon include, but are not limited to, Nextera XT DNA Library Preparation Kit (Illumina, Inc.).

Another method involves fragmentation of template DNA (e.g., full-length DNA), end repair of the fragmented DNA and/or adenylation of an end of the fragmented DNA or end-repaired fragmented DNA (also referred to as "A addition"), addition of adapter sequences to the end-repaired and/or end-adenylated DNA fragments by ligation with T4 Ligase or the like, and nucleic acid amplification of a library composed of the DNA fragments having the adapters added thereto. Since the adapter sequences have sequences necessary for next-generation sequencing added thereto, sequence analysis can be performed (this method may be referred to as a "ligation method"). The sequences and lengths of the adapters are not particularly limited, and the adapters may be, for example, Y-adapters, stem-loop adapters, or the like. Examples of commercially available kits using a ligation method include, but are not limited to, GenNext(R) NGS Library Prep Kit (Toyobo Co., Ltd.) and KAPA HyperPrep Kit (KAPA).

In one embodiment, the method for preparing the library is preferably a transposon method.

Conventionally, it is common to purify a double-stranded cDNA solution before library preparation to remove primers, adapters, dNTPs, and salts from the solution. The term "purification" as used herein refers to separating double-stranded cDNA from contaminants such as the primers contained in a solution or the like. Known purification methods include a method using magnetic beads, a method using a column, a method using phenol or phenol/chloroform, and a method using protein aggregation action. The present invention is characterized by preparing a library using unpurified double-stranded cDNA that has not undergone such a purification step. Note that the action of, for example, decomposing contaminants in the double-stranded cDNA solution synthesized in step (b) by heating or other methods does not correspond to purification in the present invention. In the present invention, the "unpurified double-stranded cDNA" is, for example, the double-stranded cDNA synthesized in step (b) (including the double-stranded cDNA in the form of a solution) itself or heated, the double-stranded cDNA diluted with water, a buffer, or other solvents, or one obtained by drying and concentrating the double-stranded cDNA solution. However, the unpurified double-stranded cDNA is not limited to these as long as it has not undergone a purification step. In the present invention, a sufficient library yield can be obtained even if purification of the double-stranded cDNA solution is omitted. In the present invention, the library concentration can be increased. The library concentration may be, for example, 10 nM or more, 15 nM or more, 20 nM or more, 25 nM or more, 30 nM or more, 35 nM or more, or 40 nM or more. In the present invention, the amount of adapter dimers in the library can be reduced. For example, when the library is measured by MultiNA (Shimadzu Corporation), the intensity of the peak in the region indicating adapter dimer formation, which is present in the region of 100 bp or more and less than 150 bp, can be 90% or less, 80% or less, 70% or less, 60% or less, or 50% or less of the maximum intensity in the region indicating a library yield (the region in the range of about 150 bp or more and 5000 bp or less).

Examples

The present invention is described in more detail with reference to Examples. However, the present invention is not limited to these Examples.

Example 1: Effect of Concentration of Sodium Chloride on Yield

In this Example, the following experiment was conducted to investigate whether the concentration of sodium chloride affects a library yield.

RNA was diluted, single-stranded cDNA was synthesized by the RT-RamDA method, and double-stranded cDNA was synthesized using a Klenow fragment. Library preparation was then performed using Nextera XT DNA Library Preparation Kit (Illumina, Inc.), which uses a transposon method, and the library concentration was measured with MultiNA (Shimadzu Corporation). The procedure was in accordance with the instructions of the manufacturers.

The nucleic acid fragment sample used in this Example was 10 pg of RNA purified from NIH3T3 cells using RNeasy Mini Kit (Qiagen). 1 ng/μL of the RNA was diluted with the RNA diluent shown in Table 1 to prepare 10 pg/μL of an RNA solution.

1 μL of the RNA solution was subjected to heat treatment at 70°C for 1 minute and 30 seconds. Subsequently, 2 μL of the RT-RamDA reaction solution shown in Table 2 was added, and RT-RamDA reaction was performed in 3 μL at 25°C for 10 minutes, at 30°C for 10 minutes, at 37°C for 30 minutes, at 50°C for 5 minutes, and at 98°C for 5 minutes. After the RT-RamDA reaction, 2 μL of each of the double-strand synthesis reaction solutions shown in Table 3 was individually added, and double-strand synthesis was performed in 5 μL at 16°C for 60 minutes, followed by heating (inactivation) at 80°C for 15 minutes. Thereafter, library preparation was performed using the unpurification protocol shown in Table 4, and the library concentration was measured with MultiNA. Table 5 shows the results.

Table 1

| Component | |
|---|---|
| Proteinase K (Promega Corporation) | 0.003 mg/mL (corresponding to 3.6 to 4.2 U/mL) |
| Polyinosinic acid potassium salt (ng/pL) | 3 |
| NP-40 (%) | 0.3 |
| RNase inhibitor (RNase Inhibitor produced by Toyobo Co., Ltd.) (U/$\mu$L) | 0.6 |

Table 2

| Concentration | Component |
|---|---|
| 100 mM | Tris-HCl (pH8.0) |
| 10 mM | Magnesium chloride |
| 50 mM | Potassium chloride |
| 1 mM | d A T P |
| 1 mm | d T T P |
| 1 mM | d G T P |
| 1 mM | d C T P |
| 0.4 $\mu$M | Oligo(dT)18 primer |
| 4 $\mu$M | NSR primer (hexamer) |
| 0.375U/$\mu$L | Reverse transcriptase (ReverTra Ace produced by Toyobo Co., Ltd.) |
| 0.375$U$/$\mu$L | RNase inhibitor (RNase Inhibitor produced by Toyobo Co., Ltd.) |
| 0.075U/$\mu$L | DNase I (DNase I produced by Thermo Fisher Scientific) |
| 50ng/$\mu$L | T4 gene 32 protein |

Table 3

| Condition | 1 | 2 | 3 | 4 |
|---|---|---|---|---|
| Tris-HCl (pH8. 0) (mM) | 6 | 6 | 24 | 24 |
| Magnesium chloride (mM) | 6 | 6 | 24 | 24 |
| Dithiothreitol (mM) | 0.6 | 0.6 | 2.4 | 2.4 |
| d AT P (mM) | 0.15 | 0.15 | 0.6 | 0.6 |
| d TTP (mM) | 0.15 | 0.15 | 0.6 | 0.6 |
| d GTP (mM) | 0.15 | 0.15 | 0.6 | 0.6 |
| d CTP (mM) | 0.15 | 0.15 | 0.6 | 0.6 |
| NSR primer (hexamer) ($\mu$M) | 20 | 20 | 20 | 20 |
| Klenow fragment (NEB) (U/pL) | 0.4 | 0.4 | 0.4 | 0.4 |
| Sodium chloride concentration (mM) | 30 | 120 | 120 | 30 |

NSR primer (hexamer) was synthesized using Sigma Custom Oligo and mixed to have the primer composition described in Ozsolak et al., Digital transcriptome profiling from attomole-level RNA samples, Genome Research, Vol. 20, 2010, pp. 519-525, for use.

Table 4

| Unpurification protocol |
|---|
| 1. Add 2.2 $\mu$L of TD Buffer to 1 $\mu$l of a double-strand synthesis solution. |
| 2. Add 1 $\mu$L of Amplicon Tagment Mix (ATM) and perform treatment at 55° C for 10 minutes. |
| 3. Add 1 $\mu$L of Neutralize Tagment Buffer (NT). |
| 4. Add 3 $\mu$L of Nextera PCR Master Mix (NPM), 1 $\mu$L of Index Primer N716. and 1 $\mu$L of Index Primer S513. |
| 5. Perform PCR reaction in the following cycle.<br>72°C          3min |

(continued)

| Unpurification protocol | | |
|---|---|---|
| 95°C | 30sec | |
| 95°C | 10sec | |
| 55°C | 30sec | ×17 |
| 72°C | 30sec | |
| 72°C | 5min. | |
| 6. Add 10 μL of AMPure beads to 10.2 μL of the PCR reaction solution. | | |
| 7. Set the resultant on a magnetic stand and allow it to stand. | | |
| 8. Wash the beads with 80% ethanol. | | |
| 9. Perform elution with 10 mM Tris-HCl (pH of 8.0; 20 μl). | | |
| TD, ATM. NT, NPM are contained in Nextera XT DNA Library Preparation Kit (Illumina, Inc.), and the Index Primers are contained in Nextera XT Index Kit v2 Set D (Illumina, Inc.). | | |

Table 5

| Condition | 1 | 2 | 3 | 4 |
|---|---|---|---|---|
| Chloride concentration (mM) at the time of synthesizing double-stranded cDNA | 38.4 | 74.4 | 81.6 | 45.6 |
| Sodium chloride concentration (mM) at the time of synthesizing double-stranded cDNA | 12 | 48 | 48 | 12 |
| Library yield (mM) | 47.6 | 31.0 | 26.7 | 40.3 |

When the chloride concentration was 50 mM or less (in particular, 40 mM or less), and the sodium chloride concentration was especially 12 mM or less, the yield increased, i.e., sufficient library yields were obtained.

Example 2: Effect of Amounts of Polyinosinic Acid Potassium Salt and Proteinase K Added on Yield

In this Example, the following experiment was conducted to investigate whether the amounts of polyinosinic acid potassium salt and proteinase K added affect a library yield.

RNA was diluted, single-stranded cDNA was synthesized by the RT-RamDA method, and double-stranded cDNA was synthesized using a Klenow fragment. Library preparation was then performed using Nextera XT DNA Library Preparation Kit (Illumina, Inc.), and the library concentration was measured with MultiNA (Shimadzu Corporation). The procedure was in accordance with the instructions of the manufacturers.

The nucleic acid fragment sample used in this Example was 10 pg of RNA purified from NIH3T3 cells using RNeasy Mini Kit (Qiagen). 1 ng/μL of the RNA was diluted with each of the RNA diluents shown in Table 6 to prepare RNA solutions (each 10 pg/μL).

1 μL of each RNA solution was subjected to heat treatment at 70°C for 1 minute and 30 seconds. Subsequently, 2 μL of the RT-RamDA reaction solution shown in Table 7 was added, and RT-RamDA reaction was performed in 3 μL at 25°C for 10 minutes, at 30°C for 10 minutes, at 37°C for 30 minutes, at 50°C for 5 minutes, and at 98°C for 5 minutes. After the RT-RamDA reaction, 2 μL of the double-strand synthesis reaction solution shown in Table 8 was added, and double-strand synthesis was performed in 5 μL at 16°C for 60 minutes, followed by heating (inactivation) at 80°C for 15 minutes. Thereafter, library preparation was performed using the unpurification protocol shown in Table 9, and the library concentration was measured with MultiNA. Table 10 shows the results.

Table 6

| Condition | 1 | 2 | 3 | 4 | 5 |
|---|---|---|---|---|---|
| Proteinase K (Promega Corporation) | 0.0015 mg/mL (corresponding to 1.8 to 2.1 U/ mL) | 0.003 mg/mL (corresponding to 3.6 to 4.2 U/ mL) | 0.006 mg/mL (corresponding to 7.2 to 8.4 U/ mL) | 0.003 mg/mL (corresponding to 3.6 to 4.2 U/ mL) | 0.003 mg/mL (corresponding to 3.6 to 4.2 U/ mL) |

(continued)

| Condition | 1 | 2 | 3 | 4 | 5 |
|---|---|---|---|---|---|
| Polyinosinic acid potassium salt (ng/L) | 3 | 3 | 3 | 1.5 | 6 |
| NP-40 (%) | 0.3 | 0.3 | 0.3 | 0.3 | 0.3 |
| RNase inhibitor (RNase Inhibitor produced by Toyobo Co., Ltd.) (U/$\mu$L) | 0.6 | 0.6 | 0.6 | 0.6 | 0.6 |

Table 7

| Concentration | Component |
|---|---|
| 100mM | Tris-HCl (pH8.0) |
| 10mM | Magnesium chloride |
| 50mM | Potassium chloride |
| 1 mM | dATP |
| 1 mM | dTTP |
| 1 mM | dGTP |
| 1 mM | dCTP |
| 0.4 $\mu$M | Oligo(dT)18 primer |
| 4 $\mu$M | NSR primer (hexamer) |
| 0.375 U/$\mu$L | Reverse transcriptase (ReverTra Ace produced by Toyobo Co., Ltd.) |
| 0.375U/$\mu$L | RNase inhibitor (RNase Inhibitor produced by Toyobo Co., Ltd.) |
| 0.075U/$\mu$L | DNase I (DNase I produced by Thermo Fisher Scientific) |
| 50ng/$\mu$L | T4 gene 32 protein |

Table 8

| Concentration | Component |
|---|---|
| 6 mM | Tris-HCl (pH8.0) |
| 30 mM | Sodium chloride |
| 6 mM | Magnesium chloride |
| 0.6 mM | Dithiothreitol |
| 0.15 mM | dATP |
| 0.15 mM | dTTP |
| 0.15 mM | dGTP |
| 0.15 mM | dCTP |
| 20 $\mu$M | NSR primer (hexamer) |
| 0.4U/$\mu$L | Klenow fragment (NEB) |

Table 9

| Unpurification protocol |
| --- |
| 1. Add 2.2 $\mu$L of TD Buffer to 1 $\mu$l of a double-strand synthesis solution. |
| 2. Add 1 $\mu$L of Amplicon Tagment Mix (ATM) and perform treatment at 55° C for 10 minutes. |
| 3. Add 1 $\mu$L of Neutralize Tagment Buffer (NT). |
| 4. Add 3 $\mu$L of Nextera PCR Master Mix (NPM), 1 $\mu$L of Index Primer N716, and 1 $\mu$L of Index Primer S513. |
| 5. Perform PCR reaction in the following cycle. 72°C 3min / 95°C 30sec / 95°C 10sec / 55°C 30sec / 72°C 30sec ×17 / 72°C 5min |
| 6. Add 10 $\mu$L of AMPure beads to 10.2 $\mu$L of the PCR reaction solution. |
| 7. Set the resultant on a magnetic stand and allow it to stand. |
| 8. Wash the beads with 80% ethanol. |
| 9. Perform elution with 10 mM Tris-HCl (pH of 8.0; 20 $\mu$l). |

Table 10

| Condition (during the step of synthesizing single-stranded cDNA) | 1 | 2 | 3 | 4 | 5 |
| --- | --- | --- | --- | --- | --- |
| Proteinase K concentration | 0.0005 mg/mL (corresponding to 0.6 to 0.7 U/mL) | 0.001 mg/mL (corresponding to 1.2 to 1.4 U/mL) | 0.002 mg/mL (corresponding to 2.4 to 2.8 U/mL) | 0.001 mg/mL (corresponding to 1.2 to 1.4 U/mL) | 0.001 mg/mL (corresponding to 1.2 to 1.4 U/mL) |
| Polyinosinic acid potassium salt (ng/L) | 1 | 1 | 1 | 0.5 | 2 |
| Library concentration (nM) | 20.9 | 33.3 | 28.1 | 17.5 | 34.7 |

When the proteinase K concentration was about 1 U (1.2 to 1.4 U)/mL (= 0.001 mg/mL) or more, the yield increased, and when the polyinosinic acid potassium salt concentration was 1 ng/$\mu$L or more, the yield also increased, i.e., sufficient library yields were obtained.

Example 3. Yield Improvement in Unpurification Protocol by Addition of Polyinosinic Acid Potassium Salt

In this Example, the following experiment was conducted to investigate whether addition of polyinosinic acid potassium salt affects a library yield.

Cells were lysed, single-stranded cDNA was synthesized by the RT-RamDA method, and double-stranded cDNA was synthesized using a Klenow fragment. Library preparation was then performed using Nextera XT DNA Library Preparation Kit (Illumina, Inc.), and the library concentration was measured with MultiNA (Shimadzu Corporation). The procedure was in accordance with the instructions of the manufacturers.

Specifically, the following method was used.

The cell sample used in this Example was prepared to have the following composition. NIH3T3 cells were reacted at 37°C for 2 minutes using a trypsin solution (Nacalai Tesque, Inc.) to be dissociated into single cells. After the dissociation, the reaction was immediately stopped by replacement with PBS(-). Cells that were negative for PI, which is a dead cell fluorescence marker dye, were defined as a living cell fraction using a FACSMelody Cell Sorter (BD). From this fraction, 120 cells were placed in 3 $\mu$L of the lysis buffer shown in Table 11. Immediately after that, the cells were centrifuged and stored at -80°C. When the cells were used for RT-RamDA reaction, 117 $\mu$L of the lysis buffer was added after the cells were thawed, and 1 $\mu$L of the cell lysate was used as a 1-cell lysate.

1 $\mu$L of the 1-cell lysate was subjected to heat treatment at 70°C for 1 minute and 30 seconds. Subsequently, 2 $\mu$L of the RT-RamDA reaction solution shown in Table 12 was added, and RT-RamDA reaction was performed in 3 $\mu$L at 25°C for 10 minutes, at 30°C for 10 minutes, at 37°C for 30 minutes, at 50°C for 5 minutes, and at 98°C for 5 minutes. After the RT-RamDA reaction, 2 $\mu$L of the double-strand synthesis reaction solution shown in Table 13 was added, and double-strand synthesis was performed in 5 $\mu$L at 16°C for 60 minutes, followed by heating (inactivation) at 80°C for 15 minutes. Thereafter, library preparation was performed using the purification and unpurification protocols shown in Table 14, and the library concentration was measured with MultiNA. Table 15 shows the results.

Table 11

| 10 % | RealTime ready Cell Lysis Buffer (Roche) |
|---|---|
| 0.3% | NP-40 (Thermo Scientific) |
| 0.6U/$\mu$L | RNase inhibitor (RNase Inhibitor produced by Toyobo Co., Ltd.) |
| 0 ng/$\mu$L (conditions 1 and 2) or 3 ng/$\mu$L (condition 3) | Polyinosinic acid potassium salt (enzymatically synthesized product produced by Sigma-Aldrich) |

Table 12

| Concentration | Component |
|---|---|
| 100 mM | Tris-HCl (pH8. 0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4 $\mu$M | Oligo(dT)18 primer |
| 4 $\mu$M | NSR primer (hexamer) |
| 0. 375U/$\mu$L | Reverse transcriptase (ReverTra Ace produced by Toyobo Co., Ltd.) |
| 0. 475U/$\mu$L | RNase inhibitor (RNase Inhibitor produced by Toyobo Co., Ltd.) |
| 0. 075U/$\mu$L | DNase I (DNase I produced by Thermo Fisher Scientific) |

(continued)

| Concentration | Component |
|---|---|
| 50 ng/$\mu$L | T4 gene 32 protein |

Table 13

| Concentration | Component |
|---|---|
| 6 mM | Tris-HCl (pH 8. 0) |
| 30 mM | Sodium chloride |
| 6 mM | Magnesium chloride |
| 0. 6 m M | Dithiothreitol |
| 0. 1 5mM | dATP |
| 0. 15mM | dTTP |
| 0. 15mM | dGTP |
| 0. 15 mM | dCTP |
| 2 0 $\mu$M | NSR primer (hexamer) |
| 0. 4 U/$\mu$L | Klenow fragment (NEB) |

Table 14

| Purification protocol (condition 1) | Unpurification protocol (conditions 2 and 3) |
|---|---|
| 1-1. Add 15 $\mu$l of AMpure beads (Beckman Coulter, Inc.) to 5 $\mu$l of a double-strand synthesis solution. | 1. Add 2.2 $\mu$L of TD Buffer to 1 $\mu$l of a double-strand synthesis solution. |
| 1-2. Set the resultant on a magnetic stand and allow it to stand. | |
| 1-3. Wash the beads with 80% ethanol. | |
| 1-4. Perform elution with 3 $\mu$l of Tagment DNA Buffer (TD) diluted to 2/3. | |
| 2. Add 1 $\mu$L of Amplicon Tagment Mix (ATM) and perform treatment at 55°C for 10 minutes. ||
| 3. Add 1 $\mu$L of Neutralize Tagment Buffer (NT). ||
| 4. Add 3 $\mu$L of Nextera PCR Master Mix (NPM), 1 $\mu$L of Index Primer N716, and 1 $\mu$L of Index Primer S513. ||
| 5. Perform PCR reaction in the following cycle.<br>72°C    3min<br>95°C    30sec<br>95°C    10sec ⎫<br>55°C    30sec ⎬ ×17<br>72°C    30sec ⎭<br>72°C    5min ||
| 6. Add 10 $\mu$L of AMpure beads to 10.2 $\mu$L of the PCR reaction solution. ||
| 7. Set the resultant on a magnetic stand and allow it to stand. ||
| 8. Wash the beads with 80% ethanol. ||

(continued)

| Purification protocol (condition 1) | Unpurification protocol (conditions 2 and 3) |
|---|---|
| 9. Perform elution with 10 mM Tris-HCl (pH of 8.0; 20 μl). | |

Table 15

| Condition (during the step of synthesizing single-stranded cDNA) | 1 | 2 | 3 |
|---|---|---|---|
| Protocol | Purification | Unpurification | Unpurification |
| Polyinosinic acid potassium salt (ng/L) | 0 | 0 | 1 |
| Library concentration (nM) | 95.3 | 13.6 | 41.3 |

Addition of polyinosinic acid in the unpurification protocol increased the yield, i.e., results in a sufficient library yield. In the purification protocol in condition 1, 5 μL of double-stranded cDNA is used, whereas in the unpurification protocol in each of conditions 2 and 3, 1 μL of double-stranded cDNA, which is 1/5 of that amount used in the purification protocol, is used for library preparation. However, in particular, in condition 3, the library yield was increased to about 1/2 of that in condition 1. The results show that the present invention enables more efficient library preparation.

Example 4: Evaluation of Amount of Template RNA

In this Example, the following experiment was conducted to investigate the amount of template RNA required for library preparation in the present invention.

RNA was diluted, single-stranded cDNA was synthesized by the RT-RamDA method or ordinary reverse transcription method (RT method), and double-stranded cDNA was synthesized using a Klenow fragment. Library preparation was then performed using Nextera XT DNA Library Preparation Kit (Illumina, Inc.), and the library concentration was measured with MultiNA (Shimadzu Corporation). The procedure was in accordance with the instructions of the manufacturers.

The nucleic acid fragment sample used in this Example was mouse ES cell total RNA (Unitech, Inc.). 1 ng/μL of the RNA was diluted with each of the RNA diluents shown in Table 16 to prepare RNA solutions (each 10 pg/μL; RNA template amount: 10 pg).

1 μL of each RNA solution was subjected to heat treatment at 70°C for 1 minute and 30 seconds. Subsequently, 2 μL of the RT-RamDA reaction solution shown in Table 17 was added in conditions 1 and 2, and 2 μL of the RT reaction solution shown in Table 18 was added in condition 3. RT-RamDA reaction or RT reaction was performed in 3 μL at 25°C for 10 minutes, at 30°C for 10 minutes, at 37°C for 30 minutes, at 50°C for 5 minutes, and at 98°C for 5 minutes. After the RT-RamDA reaction or RT reaction, 2 μL of the double-strand synthesis reaction solution shown in Table 19 was added, and double-strand synthesis was performed in 5 μL at 16°C for 60 minutes, followed by inactivation at 80°C for 15 minutes. Thereafter, library preparation was performed using the unpurification protocol shown in Table 20, and the library concentration was measured with MultiNA. Table 21 shows the results.

Table 16

| Condition | 1 | 2 | 3 |
|---|---|---|---|
| Proteinase K (Promega Corporation) | 0 | 0.003 mg/mL (corresponding to 3.6 to 4.2 U/mL) | 0.003 mg/mL (corresponding to 3.6 to 4.2 U/mL) |
| Polyinosinic acid potassium salt (ng/L) | 0 | 3 | 3 |
| NP-40 (%) | 0.3 | 0.3 | 0.3 |
| RNase inhibitor (U/μL) | 0.6 | 0.6 | 0.6 |

Table 17

| Concentration | Component |
|---|---|
| 100 mM | Tris-HCl (pH8. 0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4 $\mu$M | Oligo(dT)18 primer |
| 4 $\mu$M | NSR primer (hexamer) |
| 0. 375U/$\mu$L | Reverse transcriptase (ReverTra Ace produced by Toyobo Co., Ltd.) |
| 0. 375U/$\mu$L | RNase inhibitor (RNase Inhibitor produced by Toyobo Co., Ltd.) |
| 0. 075U/$\mu$L | DNase I (DNase I produced by Thermo Fisher Scientific) |
| 50 ng/$\mu$L | T4 gene 32 protein |

Table 18

| Concentration | Component |
|---|---|
| 100 mM | Tris-HCl (pH8. 0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0. 4 $\mu$M | Oligo(dT)18 primer |
| 4 $\mu$M | NSR primer (hexamer) |
| 0. 375U/$\mu$L | Reverse transcriptase (ReverTra Ace produced by Toyobo Co., Ltd.) |
| 0. 375U/$\mu$L | RNase inhibitor (RNase Inhibitor produced by Toyobo Co., Ltd.) |

Table 19

| Concentration | Component |
|---|---|
| 6 mM | Tris-HCl (pH 8.0) |
| 30 mM | Sodium chloride |
| 6 mM | Magnesium chloride |
| 0.6 mM | Dithiothreitol |
| 0.15 mM | dATP |
| 0.15 mM | dTTP |
| 0.15 mM | dGTP |

(continued)

| Concentration | Component |
|---|---|
| 0.15 mM | dCTP |
| 20 $\mu$M | NSR primer (hexamer) |
| 0.4 U/$\mu$L | Klenow fragment (NEB) |

Table 20

| Unpurification protocol |
|---|
| 1. Add 2.2 $\mu$L of TD Buffer to 1 $\mu$l of a double-strand synthesis solution. |
| 2. Add 1 $\mu$L of Amplicon Tagment Mix (ATM) and perform treatment at 55° C for 10 minutes. |
| 3. Add 1 $\mu$L of Neutralize Tagment Buffer (NT). |
| 4. Add 3 $\mu$L of Nextera PCR Master Mix (NPM), 1 $\mu$L of Index Primer N716, and 1 $\mu$L of Index Primer S513. |
| 5. Perform PCR reaction in the following cycle.<br>72°C 3min<br>95°C 30sec<br>95°G 10sec<br>55°C 30sec<br>72'C 30sec<br>$\left.\begin{array}{l}\quad\end{array}\right\} \times 17$<br>72°C 5min |
| 6. Add 10 $\mu$L of AMPure beads to 10.2 $\mu$L of the PCR reaction solution. |
| 7. Set the resultant on a magnetic stand and allow it to stand. |
| 8. Wash the beads with 80% ethanol. |
| 9. Perform elution with 10 mM Tris-HCl (pH of 8.0; 20 $\mu$l). |

Table 21

| Condition (during the of synthesizing single-stranded cDNA) | 1 | 2 | 3 |
|---|---|---|---|
| Single-stranded cDNA synthesis method | RT-RamDA method | RT-RamDA method | RT method |
| Proteinase K concentration | 0 | 0.001 mg/mL (corresponding to 1.2 to 1.4 U/mL) | 0.001 mg/mL (corresponding to 1.2 to 1.4 U/mL) |
| Polyinosinic acid potassium salt (ng/$\mu$L) | 0 | 1 | 1 |
| Library concentration (nM) | 27.7 | 54.3 | 9.7 |

Sufficient yields were obtained from 10 pg of total RNA in conditions 1 and 3. Furthermore, the yield was higher in condition 2 than in condition 1, and it is believed that the use of proteinase K and polyinosinic acid potassium salt increased the yield. The results of this Example show that the present invention enables a sufficient amount of library to be prepared without a purification step when the amount of template RNA used is at least 10 pg.

Example 5: Comparison of Inactivation Temperatures in Double-strand Synthesis

In this Example, the following experiment was conducted to investigate the effect of the inactivation temperature after double-strand synthesis on a yield.

RNA was diluted, single-stranded cDNA was synthesized by the RT-RamDA method, and double-stranded cDNA was synthesized using a Klenow fragment, followed by inactivation by heating at 70°C or 80°C. Library preparation was then performed using Nextera XT DNA Library Preparation Kit (Illumina, Inc.), and the library concentration was measured with MultiNA (Shimadzu Corporation). The procedure was in accordance with the instructions of the manufacturers.

The nucleic acid fragment sample used in this Example was mouse ES cell total RNA. 1 ng/μL of the RNA was diluted with the RNA diluent shown in Table 22 to prepare 10 pg/μL of an RNA solution (RNA template amount: 10 pg).

1 μL of the RNA solution was subjected to heat treatment at 70°C for 1 minute and 30 seconds. Subsequently, 2 μL of the RT-RamDA reaction solution shown in Table 23 was added, and RT-RamDA reaction was performed in 3 μL at 25°C for 10 minutes, at 30°C for 10 minutes, at 37°C for 30 minutes, at 50°C for 5 minutes, and at 98°C for 5 minutes. After the RT-RamDA reaction, 2 μL of the double-strand synthesis reaction solution shown in Table 24 was added, and double-strand synthesis was performed in 5 μL at 16°C for 60 minutes, followed by heating at 70°C for 10 minutes (condition 1) or at 80°C for 15 minutes (condition 2) for inactivation (n = 2). Thereafter, library preparation was performed using the unpurification protocol shown in Table 25, and the library concentration was measured with MultiNA. Table 26 shows the results. Representative measurement results are also shown in Fig. 1 (condition 1 is the result of (1), and condition 2 is the result of (3)).

Table 22

| Proteinase K (Promega Corporation) | 0.003 mg/mL (corresponding to 3.6 to 4.2 U/mL) |
| --- | --- |
| Polyinosinic acid potassium salt (ng/μL) | 3 |
| NP-40 (%) | 0.3 |
| RNase inhibitor (U/μL) | 0.6 |

Table 23

| Concentration | Component |
| --- | --- |
| 100 nM | Tris-HCl (pH8.0) |
| 10 mM | Magnesium chloride |
| 50 mM | Potassium chloride |
| 1 mM | dATP |
| 1 mM | dTTP |
| 1 mM | dGTP |
| 1 mM | dCTP |
| 0.4 μM | Oligo(dT)18 primer |
| 4 μM | NSR primer (hexamer) |
| 0.375U/μL | Reverse transcriptase (ReverTra Ace produced by Toyobo Co., Ltd.) |
| 0.375U/μL | RNase inhibitor (RNase Inhibitor produced by Toyobo Co., Ltd.) |
| 0.075U/μL | DNase I (DNase I produced by Thermo Fisher Scientific) |
| 50 ng/μL | T4 gene 32 protein |

Table 24

| Concentration | Component |
|---|---|
| 6 mM | Tris-HCl (pH8.0) |
| 30 mM | Sodium chloride |
| 6 mM | Magnesium chloride |
| 0.6 mM | Dithiothreitol |
| 0.15mM | dATP |
| 0.15mM | dTTP |
| 0.15mM | dGTP |
| 0.15mM | dCTP |
| 20 $\mu$M | NSR primer (hexamer) |
| 0.4U/$\mu$L | Klenow fragment (NEB) |

Table 25

| Unpurification protocol |
|---|
| 1. Add 2.2 $\mu$L of TD Buffer to 1 $\mu$l of a double-strand synthesis solution. |
| 2. Add 1 $\mu$L of Amplicon Tagment Mix (ATM) and perform treatment at 55° C for 10 minutes. |
| 3. Add 1 $\mu$L of Neutralize Tagment Buffer (NT). |
| 4. Add 3 $\mu$L of Nextera PCR Master Mix (NPM), 1 $\mu$L of Index Primer N716, and 1 $\mu$L of Index Primer S513. |
| 5. Perform PCR reaction in the following cycle. <br> 72°C 3min <br> 95°C 30sec <br> 95°C 10sec <br> 55°C 30sec } ×17 <br> 72°C 30sec <br><br> 72°C 5min |
| 6. Add 10 $\mu$L of AMPure beads to 10.2 $\mu$L of the PCR reaction solution. |
| 7. Set the resultant on a magnetic stand and allow it to stand. |
| 8. Wash the beads with 80% ethanol. |
| 9. Perform elution with 10 mM Tris-HCl (pH of 8.0; 20 $\mu$l). |

Table 26

| Inactivation condition after double-strand synthesis | Condition 1 (70° C for 10 minutes) | | Condition 2. (80° C for 15 minutes) | |
|---|---|---|---|---|
| | (1) | (2) | (3) | (4) |
| Library concentration (nM) | 23.2 | 19.7 | 44.0 | 46.7 |

As shown in the results of Table 26 and Fig. 1, the yields in condition 2, in which inactivation was performed at 80°C, were higher than those in condition 1, in which inactivation was performed at 70°C. Furthermore, as shown in Fig. 1, in condition 2, in which inactivation was performed at 80°C, the peak (arrow) indicating the formation of adapter dimers was significantly decreased, which shows that the formation of adapter dimers was significantly suppressed. The results of this Example show that inactivation by heat treatment at 80°C or higher after double-strand synthesis more effectively suppresses dimer formation and improves the library yield.

Example 6: Evaluation of Chloride in Step (b)

In this Example, the following experiment was conducted to investigate the type etc. of chloride in step (b).

RNA was diluted, single-stranded cDNA was synthesized by the RT-RamDA method, and double-stranded cDNA was synthesized using a Klenow fragment. Library preparation was then performed using Nextera XT DNA Library Preparation Kit (Illumina, Inc.), and the library concentration was measured with MultiNA (Shimadzu Corporation). The procedure was in accordance with the instructions of the manufacturers.

The nucleic acid fragment sample used in this Example was total RNA purified from human K562 cells using RNeasy mini kit (Qiagen). 1 ng/$\mu$L of the RNA was diluted with the RNA diluent shown in Table 1 of Example 1 to prepare 10 pg/$\mu$L of an RNA solution (RNA template amount: 10 pg).

1 $\mu$L of the RNA solution was subjected to heat treatment at 70°C for 1 minute and 30 seconds. After the heat treatment, 2 $\mu$L of the RT-RamDA reaction solution shown in Table 2 of Example 1 was added, and RT-RamDA reaction was performed in 3 $\mu$L at 25°C for 10 minutes, at 30°C for 10 minutes, at 37°C for 30 minutes, at 50°C for 5 minutes, and at 98°C for 5 minutes. After the RT-RamDA reaction, 2 $\mu$L of each of the double-strand synthesis reaction solutions shown in Table 27 was individually added, and double-strand synthesis was performed in 5 $\mu$L at 16°C for 60 minutes, followed by inactivation at 80°C for 15 minutes. Thereafter, library preparation was performed using the unpurification protocol shown in Table 4 of Example 1, and the library concentration was measured with MultiNA. Table 28 shows the results.

Table 27

| Condition 1 | Condition 2 |
|---|---|
| 6 mM Tris-HCl (pH8.0) | 6 mM Tris-HCl (pH8.0) |
| 30 mM sodium chloride | 30 mM potassium chloride |
| 6 mM magnesium chloride | 6 mM magnesium chloride |
| 0.6 mM dithiothreitol | 0.6 mM dithiothreitol |
| 0.15 mM dATP | 0.15 nM dATP |
| 0.15 mM dTTP | 0.15 mM dTTP |
| 0.15 mM dGTP | 0.15 mM dGTP |
| 0.15 mM dCTP | 0.15 mM dCTP |
| 20 $\mu$M NSR primer (hexamer) | 20 $\mu$M NSR primer (hexamer) |
| 0.4 U/$\mu$L Klenow fragment (NEB) | 0.4 U/$\mu$L Klenow fragment (NEB) |

Table 28

| Condition | 1 | 2 |
|---|---|---|
| Single-stranded cDNA synthesis method | RT-RamDA method | RT-RamDA method |

(continued)

| Condition | 1 | 2 |
| --- | --- | --- |
| Chloride concentration (mM) at the time of synthesizing double-stranded cDNA | 38.4 | 38.4 |
| Sodium chloride concentration (mM) at the time of synthesizing double-stranded cDNA | 12 | 0 |
| Library concentration (nM) | 41.3 | 43.2 |

Sufficient yields were obtained from 10 pg of total RNA in conditions 1 and 2. There was little difference in yield between condition 1 and condition 2. The results show that the present invention enables preparation of a sufficient amount of library even when potassium chloride is added in place of sodium chloride in step (b).

**Claims**

1. A method for preparing a library, comprising the following steps (a), (b), and (c):

   (a) synthesizing single-stranded cDNA from 10 pg or more of template RNA;
   (b) synthesizing double-stranded cDNA from the single-stranded cDNA; and
   (c) preparing a library using the double-stranded cDNA that is unpurified.

2. The method according to claim 1, wherein step (b) is performed in the presence of 12 mM or less of sodium chloride.

3. The method according to claim 1, wherein step (a) is performed in the presence of 1 ng/μL or more of a nucleic acid polymer and/or 1 U/mL or more of a proteolytic enzyme.

4. The method according to claim 1, further comprising heating the double-stranded cDNA at 80°C or higher for 10 minutes or more.

5. The method according to claim 3, wherein the nucleic acid polymer is at least one homopolymer selected from the group consisting of polyinosinic acid, polycytidylic acid, polyguanylic acid, polyadenylic acid, polythymidylic acid, polyuridylic acid, polydeoxyinosinic acid, polydeoxycytidylic acid, polydeoxyguanylic acid, polydeoxyadenylic acid, polydeoxythymidylic acid, polydeoxyuridylic acid, and salts thereof.

6. The method according to claim 3, wherein the nucleic acid polymer is at least one homopolymer selected from the group consisting of polyinosinic acid, polydeoxyinosinic acid, and salts thereof.

7. The method according to claim 3, wherein the proteolytic enzyme comprises any of proteinase K and subtilisin.

8. The method according to any one of claims 1 to 7, wherein the template RNA is RNA extracted from 1 to 1000 cells.

9. The method according to any one of claims 1 to 7, wherein the double-stranded cDNA that is unpurified of step (c) is in the form of 1 μL or less of a solution.

10. The method according to any one of claims 1 to 7,
    wherein step (a) is performed by an RT-RamDA method.

11. The method according to any one of claims 1 to 7, wherein step (b) is performed using a Klenow fragment.

12. The method according to any one of claims 1 to 7,
    wherein step (c) is performed by any of a transposon method and a ligation method.

13. The method according to any one of claims 1 to 7,
    wherein step (b) is performed in the presence of 60 mM or less of a chloride.

Fig. 1

Condition 1
(1)

Condition 2
(3)

## INTERNATIONAL SEARCH REPORT

| International application No. |
| --- |
| **PCT/JP2022/041394** |

**A. CLASSIFICATION OF SUBJECT MATTER**

***C12N 15/10***(2006.01)i; ***C12Q 1/6806***(2018.01)i; ***C40B 40/06***(2006.01)i
FI: C12N15/10 Z; C40B40/06; C12Q1/6806 Z

According to International Patent Classification (IPC) or to both national classification and IPC

**B. FIELDS SEARCHED**

Minimum documentation searched (classification system followed by classification symbols)

C12N15/10; C12Q1/6806; C40B40/06

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | HAYASHI, T. et al. Single-cell full-length total RNA sequencing uncovers dynamics of recursive splicing and enhancer RNAs. Nat. Commun. 2018, vol. 9, article number: 619, pp. 1-16 | 1-3, 5, 7, 8, 10-13 |
| | in particular, p. 2, right column, p. 12, Sequencing library preparation for C1-RamDA-seq | |
| Y | | 1-13 |
| Y | WO 2020/184551 A1 (TOYO BOSEKI) 17 September 2020 (2020-09-17) claims, paragraph [0028], examples | 1-13 |

☐ Further documents are listed in the continuation of Box C.　　☑ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **13 January 2023** | **24 January 2023** |

| Name and mailing address of the ISA/JP | Authorized officer |
| --- | --- |
| **Japan Patent Office (ISA/JP)** 3-4-3 Kasumigaseki, Chiyoda-ku, Tokyo 100-8915 Japan | |
| | Telephone No. |

Form PCT/ISA/210 (second sheet) (January 2015)

**INTERNATIONAL SEARCH REPORT**
Information on patent family members

International application No.

**PCT/JP2022/041394**

| Patent document cited in search report | Publication date (day/month/year) | Patent family member(s) | Publication date (day/month/year) |
|---|---|---|---|
| WO 2020/184551 A1 | 17 September 2020 | US 2022/0154180 A1 claims, paragraphs [0124]-[0130], examples | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2016052619 A
- US 20170275685
- US 2014178438

### Non-patent literature cited in the description

- **HAYASHI T. et al.** Single-cell full-length total RNA sequencing uncovers dynamics of recursive splicing and enhancer RNAs. *Nat. Commun.,* 2018
- **OZSOLAK et al.** Digital transcriptome profiling from attomole-level RNA samples. *Genome Research,* 2010, vol. 20, 519-525